# EUROPEAN PATENT APPLICATION

(11) **EP 4 098 181 A1**
(43) Date of publication of application: **07.12.2022**
(21) Application number: 22176792.4
(22) Date of filing: 01.06.2022
(51) Int. Cl.: A61B 5/02, A61B 5/18, G08B 21/06

(54) **EMBEDDING SENSORS IN AN AIRCRAFT CONTROL**

(30) Priority: 04.06.2021 US 202163196789 P; 17.05.2022 US 202217746596
(71) Applicant: Rockwell Collins, Inc., Cedar Rapids, IA 52498 (US)
(72) Inventor: WU, Peggy, East Hartford, CT (US); GEORGE, Christopher L., Winchester, VA (US); WITTKOP, Timothy J., Marion, IA (US); MATESSA, Michael P., Ben Lomond, CA (US); RAO, Arjun Harsha, Marion, IA (US); JOHNSON, Wade T., Cedar Rapids, IA (US)
(74) Representative: Dehns

(57) **Abstract**

An aircraft system includes an aircraft control having embedded biometric sensors (104, 106). The biometric sensors record pilot physiological data at various phases of flight for analysis and pilot monitoring. The biometric data may be recorded and correlated to flight phases to establish a base-line profile for an individual pilot or an individual flight phase for later comparison and monitoring. Certain biometric data may be used as a secondary characteristic to confirm the identity of a pilot, or to verify that a pilot is currently engaging the aircraft control.

## Description

### BACKGROUND

Pilot monitoring system are limited by the availability of biometric data. Commercial pilots typically refuse systems which have invasive sensors, however having some sense of the pilot's state allows for greater ability to alert when dangerous situations arise.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system including aircraft control embedded sensors according to an exemplary embodiment;
FIG. 2A shows a top view of an aircraft control with embedded sensors according to an exemplary embodiment;
FIG. 2B shows a side view of an aircraft control with embedded sensors according to an exemplary embodiment;
FIG. 2C shows a side view of an aircraft control with embedded sensors according to an exemplary embodiment;
FIG. 2D shows a front view of an aircraft control with embedded sensors according to an exemplary embodiment;

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

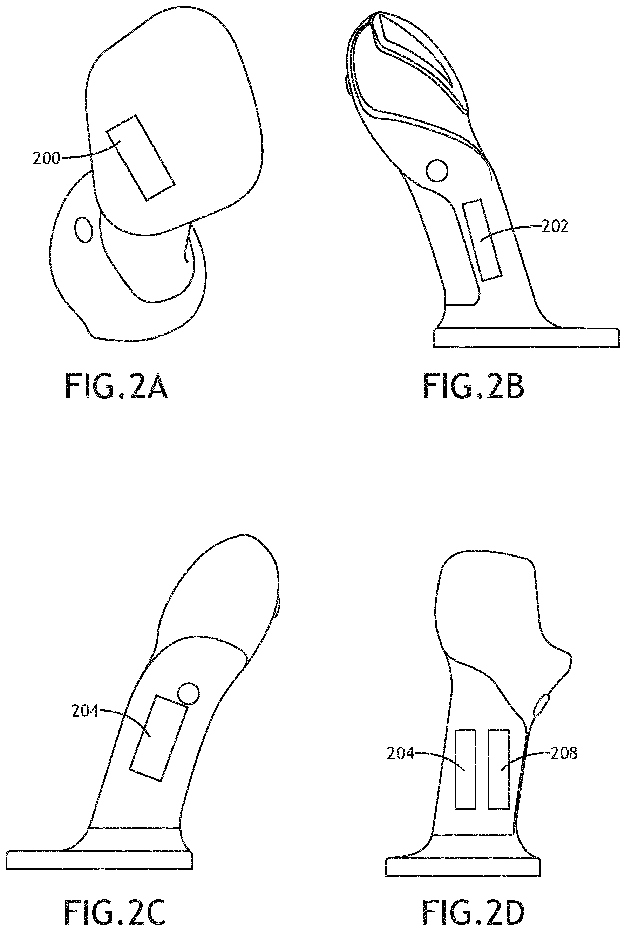

Broadly, embodiments of the inventive concepts disclosed herein are directed to an aircraft system including an aircraft control having embedded biometric sensors. The biometric sensors record pilot physiological data at various phases of flight for analysis and pilot monitoring. The biometric data may be recorded and correlated to flight phases to establish a base-line profile for an individual pilot or an individual flight phase for later comparison and monitoring. Certain biometric data may be used as a secondary characteristic to confirm the identity of a pilot, or to verify that a pilot is currently engaging the aircraft control. In the context of the present disclosure, "aircraft control" should be understood to refer to hand operated controls such as aircraft sidesticks (as shown in FIGS. 2A-2D), aircraft yoke controls, and other manually operated mechanisms such as throttle controls. While an aircraft sidestick is specifically shown, other hand operated controls are envisioned.

Embodiments of the present disclosure may be more fully understood with reference to U.S. Patent App. No. 63/196,765 ("Cognitive Battery for Return to Service") (filed June 4, 2021) and U.S. Patent App. No. 63/196,784 ("Physiological and Behavioural Methods to Assess Pilot Readiness") (filed June 4, 2021).

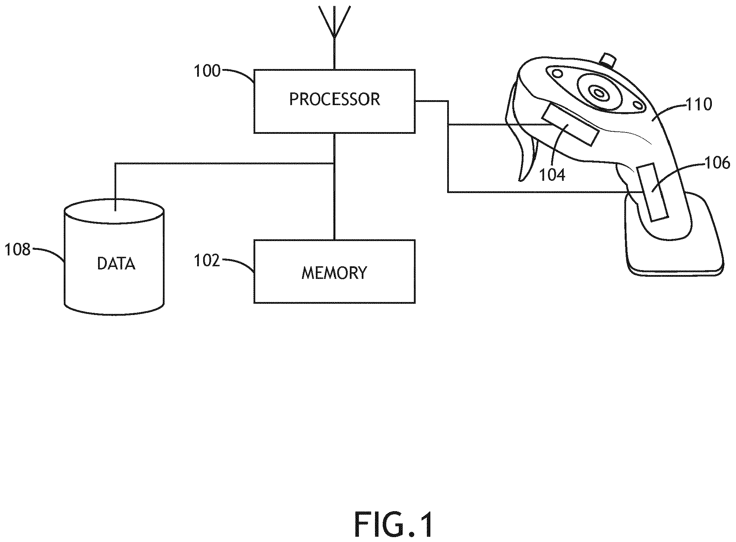

Referring to FIG. 1, a block diagram of a system including aircraft control embedded sensors 104, 106 according to an exemplary embodiment is shown. The system includes a processor 100, memory 102 for embodying processor executable code, a aircraft control 110 for performing avionics inputs, and biometric sensors 104, 106 embedded at various locations in the aircraft control 110. In at least one embodiment, the processor 100 is configured to receive data from the biometric sensors 104, 106 and store the data in a data storage element 108.

The biometric sensors 104, 106 may comprise one or more of a pulse rate sensor, an O₂ sensor, a galvanic skin response sensor, a contact electro-encephalogram sensor, a capacitive electro-cardiograph sensor, etc. The sensors 104, 106 are disposed and embedded at locations on the aircraft control 110 according to type and function. In at least one embodiment, pulse rate and O₂ sensors 104, 106 may be disposed in a front surface of the aircraft control corresponding to the pilot's fingers. Alternatively, or in addition, those sensors 104, 106 may be disposed at the pilot's thumb pad. In at least one embodiment, galvanic skin response 104, 106 may be disposed in a side surface of the aircraft control 110 corresponding to the pilot's palm.

In at least one embodiment, the processor 100 may be configured to continuously or periodically record biometric data points and correlate those data points to a known flight phase. Such data may be periodically transmitted to ground station or data repository via a datalink connection.

In at least one embodiment, the biometric sensors 104, 106 may identify when a pilot is actively gripping the aircraft control 110, usually during high workload situations like take-off and landing. Furthermore, recorded biometric data may be used to corroborate an identification of a pilot.

In at least one embodiment, the sensors 104, 106 and recorded data may be used to determine the pilot's physical and mental state. When in data communication with avionics systems via the datalink connection, the data could then be used to initiate process steps to avert a dangerous situation such as alerting the pilot, alerting the co-pilot, alerting ground control, engaging an autopilot / emergency system, etc.

Referring to FIGS. 2A-2D, views of an aircraft control with embedded sensors 200, 202, 204, 206, 208 according to various exemplary embodiments are shown. In at least one embodiment, a biometric sensor 200 is disposed at a thumbpad of the aircraft control which may be advantageous for pulse and O₂ sensors. In at least one embodiment, biometric sensors 202 may be disposed at a side surface of the aircraft control corresponding to the pilot's finger tips. In at least one embodiment, biometric sensors 204 may be disposed at a side surface of the aircraft control corresponding to the pilot's palm. In at least one embodiment, the biometric sensors 206, 208 may be disposed at a front surface corresponding to the proximal or intermediate phalanges of the pilot. In each case, disposition may be dictated by the type of sensor 200, 202, 204, 206, 208 and the contact necessary for such sensor to function properly.

Embedding sensors in the pilot controls allows pilot vitals to be monitored without relying on a pilot wearing invasive sensors. The sensor data provides additional insight to the pilot's state and can be used for better context and alerting in critical phases of flight.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the scope of the invention as defined by the claims.

## Claims

1. A computer apparatus comprising:
a plurality of biometric sensors (104, 106) disposed in an aircraft control (110);
a data storage element (108); and
at least one processor (100) in data communication with the plurality of biometric sensors, the data storage element, and a memory storing processor executable code for configuring the at least one processor to:
continuously receive biometric data from the plurality of biometric sensors;
associating each of the biometric data with a flight phase; and
storing the biometric data in the data storage element.

2. The computer apparatus of Claim 1, wherein the plurality of biometric sensors comprises a contact electro-encephalogram sensor, and/or
wherein the plurality of biometric sensors comprises capacitive electro-cardiograph electrodes, and/or
wherein the plurality of biometric sensors comprises at least one pulse sensor, and/or
wherein the plurality of biometric sensors comprises at least one O₂ sensor.

3. The computer apparatus of Claim 1 or 2, wherein the at least one processor is further configured to:
identify a high-workload flight phase; and
continuously verify that a pilot is engaging the aircraft control.

4. The computer apparatus of Claim 3, wherein the at least one processor is further configured to:
establish a datalink connection with an avionics system;
determine that the pilot has experienced a physiological event based on a deviation in the biometric data; and
issue a warning to the avionics system.

5. An aircraft control comprising:
a plurality of biometric sensors (104, 106), the plurality of biometric sensors disposed at locations in the aircraft control corresponding to locations on a pilot's hand.

6. The aircraft control of Claim 5, wherein at least one of the plurality of biometric sensors is disposed at a location corresponding to a pilot's thumbpad, and/or wherein at least one of the plurality of biometric sensors is disposed on a front surface of the aircraft control at a location corresponding to a pilot's proximal or intermediate phalanges, and/or wherein at least one of the plurality of biometric sensors is disposed at a location corresponding to a pilot's finger tips, and/or wherein at least one of the plurality of biometric sensors is disposed at a location corresponding to a pilot's palm.

7. A system comprising:
a plurality of biometric sensors (104, 106) disposed in an aircraft control (110);
a data storage element (108); and
at least one processor (109) in data communication with the plurality of biometric sensors, the data storage element, and a memory storing processor executable code for configuring the at least one processor to:
continuously receive biometric data from the plurality of biometric sensors;
associating each of the biometric data with a flight phase; and
storing the biometric data in the data storage element.

8. The system of Claim 7, wherein the at least one processor is further configured to:
establish a biometric profile of a pilot based on a stored biometric data.

9. The system of Claim 7 or 8, wherein the plurality of biometric sensors comprises a contact electro-encephalogram sensor, and/or wherein the plurality of biometric sensors comprises capacitive electro-cardiograph electrodes, and/or wherein the plurality of biometric sensors comprises at least one pulse sensor, and/or wherein the plurality of biometric sensors comprises at least one O₂ sensor.

10. The system of Claim 7, 8 or 9, wherein the at least one processor is further configured to:
identify a high-workload flight phase; and
continuously verify that a pilot is engaging the aircraft control.

11. The system of Claim 10, wherein the at least one processor is further configured to:
establish a datalink connection with an avionics system;
determine that the pilot has experienced a physiological event based on a deviation in the biometric data; and
issue a warning to the avionics system.
